# EUROPEAN PATENT APPLICATION

(11) **EP 1 097 918 A1**
(43) Date of publication of application: **09.05.2001**
(21) Application number: 99929855.7
(22) Date of filing: 15.07.1999
(51) Int. Cl.: C07C 201/16, C07C 201/08, C07D 205/12, C07C 205/45, C07C 205/58, C07D 401/10, A61K 31/44

(54) **PROCESS FOR PRODUCING 4-SUBSTITUTED-2-NITRO-FLUOROBENZENES**

(30) Priority: 15.07.1998 JP 20101598
(71) Applicant: Meiji Seika Kaisha, Ltd., Tokyo 104-8002 (JP)
(72) Inventor: MUNAKATA, Mikio, Pharmaceutical Technology Labs., Odawara-shi, Kanagawa 250-0852 (JP); SUMI, Shinjiro, Pharmaceutical Technology Labs., Odawara-shi, Kanagawa 250-0852 (JP); MURAI, Yasushi, Pharmaceutical Technology Labs., Odawara-shi, Kanagawa 250-0852 (JP); IINUMA, Katsuharu, Pharmaceutical Technology Labs., Odawara-shi, Kanagawa 250-0852 (JP)
(74) Representative: Kyle, Diana
(86) International application number: JP9903825
(87) International publication number: WO0003971

(57) **Abstract**

A process for selectively eliminating a compound of general formula (II) from a mixture of a compound of general formula (I) with the compound of general formula (II). This process comprises reacting the compound of general formula (II) with a tertiary amine and a nucleophilic agent and then washing and/or crystallizing the thus obtained compound to thereby separate the same from the mixture. A process for producing a compound represented by general formula (I) which is free from any mutagen is also provided. In these formulae, R¹ represents alkyl, alkyl carbonyl or alkoxycarbonyl; and Hal represents halogeno.

## Description

### BACKGROUND OF THE INVENTION

### Field of the Invention

The present invention relates to a process for removing 2,4-dinitrofluorobenzene produced as a by-product in nitration of 4-substituted fluorobenzene, and a process for producing 4-substituted-2-nitrofluorobenzene free from a mutagen.

### Background Art

4-Substituted-2-nitrofluorobenzenes are compounds useful as starting compounds for pharmaceuticals and agricultural chemicals. In particular, as described in Japanese Patent Laid-Open No. 510754/1996, Japanese Patent Laid-Open No. 249623/1997 and the like, 4'-fluoro-3'-nitroacetophenone is a starting compound for medicaments for the treatment of various diseases such as diabetes, adiposis, or hyperlipidemia.

On the other hand, as reported in Org. Synth. III, 653 (1955), Org. Synth., IV, 735 (1963), Org. Synth., V, 1067 (1973), nitration of an aromatic compound can be easily carried out by using a reagent such as a nitric acid, a mixed acid, or a metal nitrate. It is described in Cram, Organic Chemistry, 4th ed. P. 668 in Japanese that an ipso substitution reaction occurs for some substrates.

The nitration of a 4-substituted fluorobenzene with nitric acid, a mixed acid, a metal nitrate or the like results in the formation of 2,4-dinitrofluorobenzene as a byproduct in addition to a 4-substituted 2-nitrofluorobenzene as the target product.

2,4-Dinitrofluorobenzene is known to have strong toxicity against rats and mice (National Academy of Sciences, National Research Council 5, 17, 1953, Cancer Research 29, 179, 1969, Biochemical Journal 41, 558, 1947) or to exhibit mutagenicity at a low concentration in a mutagenic test using Salmonella typhimurium (Sangyo Igaku Japanese Journal of Industrial Health 29, 34, 1987 and Environmental Mutagenesis 5 (Suppll) 3, 1983).

### SUMMARY OF THE INVENTION

The present inventors have now found that when producing a compound represented by formula (V): 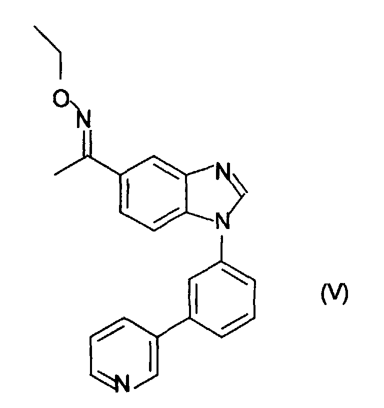 (hereinafter referred to as ME3127; see WO96/33191), which is useful for the treatment of central nervous system (CNS) disorders, a mutagen derived from 2,4-dinitrofluorobenzene is mixed with the product.

The present inventors have further found that the contamination with the mutagen is due to byproduction of 2,4-dinitrofluorobenzene and that 2,4-dinitrofluorobenzene is formed as a byproduct when 4-substituted-2-nitrofluorobenzene is produced by nitration of 4-substituted-fluorobenzene.

It is an object of the present invention to provide a process for selectively removing 2,4-dinitrofluorobenzene as a mutagen from a mixture of 4-substituted-2-nitrofluorobenzene and 2,4-dinitrofluorobenzene.

It is another object of the present invention to provide a process for producing 4-substituted-2-nitrofluorobenzene free from a mutagen on an industrial scale, in a simple manner, at high purity, and at a low cost.

It is still another object of the present invention to provide ME3127 free from a mutagen, and a process for producing the same.

According to the present invention, there is provided a process for removing a compound represented by formula (II): 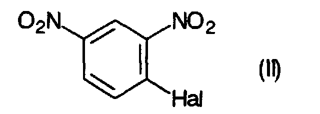 wherein Hal represents a halogen atom, from a mixture of a compound represented by formula (I): 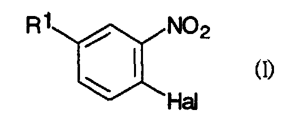 wherein R¹ represents a lower alkyl group, a lower alkylcarbonyl group, or a lower alkoxycarbonyl group, and Hal represents a halogen atom, and the compound represented by formula (II), comprising the steps of:
(a) reacting the compound represented by formula (II) with a tertiary amine and a nucleophilic reagent; and
(b) removing from the mixture a compound represented by formula (III) obtained in step (a): 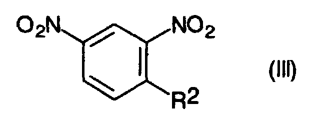 wherein R² represents a residue of the nucleophilic reagent.

According to the present invention, there is also provided a composition comprising a compound represented by formula (I), which is free from a mutagen. This composition is useful as a starting compound for pharmaceuticals and agricultural chemicals, such as ME3127.

According to the present invention, there is further provided a process for producing a compound represented by formula (I), comprising the steps of:
(c) nitrating a compound represented by formula (IV): 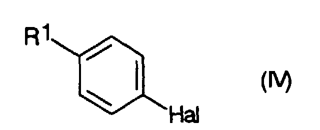 wherein R¹ and Hal are as defined in claim 1, to give a mixture containing a compound represented by formula (I) and a compound represented by formula (II); and
(d) removing the compound represented by formula (II) from the mixture of the compound represented by formula (I) and the compound represented by formula (II).

According to the present invention, there is also provided a composition comprising ME3127, which is free from a mutagen. This composition is useful as bulks of pharmaceuticals.

According to the present invention, there is further provided a process for producing ME3127, comprising the steps of:
producing a compound represented by formula (I), which is free from a mutagen;
reacting the compound represented by formula (I) with a compound represented by formula (VI): 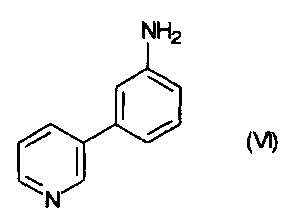 to produce a compound represented by formula (VII); 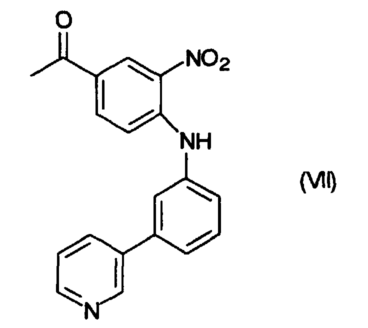
forming a benzimidazole ring in the compound represented by formula (VII); and
converting an acetyl group of formula (VII) into CH₃CH₂ON=C(CH₃)-.

### DESCRIPTION OF THE PREFERRED EMBODIMENTS

The term "lower alkyl" or "lower alkoxy" group, used herein as a group or a portion of the group, refers to a C₁-C₆, preferably a C₁-C₄ alkyl or alkoxy group.

Examples of the lower alkyl group include methyl, ethyl, n-propyl, isopropyl, n-butyl, isobutyl, and t-butyl groups.

The term "halogen" used herein refers to fluorine, chlorine, bromine, and iodine.

According to the present invention, there is provided a process for selectively removing a compound represented by formula (II) as a mutagen from a mixture of a compound represented by formula (I) and a compound represented by formula (II). This process comprises step (a) of converting only a compound represented by formula (II) into other compounds and step (b) of removing the converted compound. According to this process, a compound represented by formula (II) can be removed completely from a mixture of a compound represented by formula (I) and a compound represented by formula (II).

Step (a) can be carried out by dissolving a mixture of a compound represented by formula (I) and a compound represented by formula (II) in an organic solvent, adding a nucleophilic reagent and a tertiary amine, and reacting them. According to step (a), a compound represented by formula (II) can be converted selectively into other compounds.

Regarding the amount of the reagents to be used in the reaction, a nucleophilic reagent can be used at 15 to 200 equivalents per one mole of 2,4-dinitrofluorobenzene and a tertiary amine can be used at 15 to 100 equivalents per one mole of 2,4-dinitrofluorobenzene. More preferably, a nucleophilic reagent can be used at 30 to 60 equivalents per one mole of 2,4-dinitrofluorobenzene and a tertiary amine can be used at 30 to 60 equivalents per one mole of 2,4-dinitrofluorobenzene. An optimal temperature can be determined depending on a nucleophilic reagent to be used. The reaction may be carried out at -50 to 50 °C.

Step (b) is carried out by redissolving the reaction mixture obtained in step (a), optionally evaporated to dryness, in an organic solvent such as ethyl acetate or methylene chloride, washing the resulting solution with an aqueous sodium hydrogencarbonate solution, saline, water or the like, and, if necessary, recrystallizing from an alcohol, hydrous acetonitrile or the like. According to step (b), a compound represented by formula (III) converted from a compound represented by formula (II) can be separated from the mixture.

The term "a nucleophilic reagent" as used herein refers to secondary alcohol such as isopropyl alcohol; tertiary alcohol such as t-butyl alcohol; aromatic alcohol such as benzyl alcohol and anise alcohol; phenols and substituted phenols such as phenol and cresol; hydroxyacid such as hydroxyacetic acid, lactic acid, hydroxybutyric acid, and tartaric acid, and derivatives of hydroxyacid having a protecting group at a carboxyl group such as methyl lactate and dimethyl tartrate; and carboxylic acid such as formic acid and acetic acid. Preferably, a nucleophilic reagent is lactic acid, isopropyl alcohol, t-butyl alcohol, anise alcohol, or acetic acid.

Examples of tertiary amines include N-methyl piperazine, trimethylamine, triethylamine, diisopropylethylamine, pyridine, and N,N'-dimethylaminopyridine. Preferably, a tertiary amine is triethylamine.

When hydroxyacid (particularly, lactic acid) is used as a nucleophilic acid, a compound represented by formula (III) converted from a compound represented by formula (II) can be completely removed only by washing with an aqueous solvent (e.g. an aqueous sodium hydrogencarbonate solution). An additional by-product derived from hydroxyacid is not formed.

A "residue of a nucleophilic reagent" represented by R² can be determined depending on a nucleophilic reagent to be used.

Specifically, when alcohols, phenols and hydroxyacid are used as a nucleophilic reagent, a moiety of the nucleophilic reagent excluding a hydrogen atom from its hydroxyl group can be "a residue of a nucleophilic reagent". For example, when a nucleophilic reagent is lactic acid or lactic acid having a protected carboxyl group, R² can be -OCH(CH₃)COOR³ (R³ represents a hydrogen atom or a protecting group of a carboxyl group).

When a nucleophilic reagent to be used is carboxylic acid, a moiety of a nucleophilic reagent excluding a hydrogen atom from its carboxyl group can be "a residue of a nucleophilic reagent".

According to a preferred aspect of the present invention, there is provided a process for removing a compound represented by formula (II) from a mixture of a compound represented by formula (I) (wherein R¹ is as defined above and Hal represents fluorine) and a compound represented by formula (II) (wherein Hal represents fluorine), comprising the steps of:
(a') reacting a compound represented by formula (II) with triethylamine and lactic acid optionally protected at its carboxyl group; and
(b') separating from the mixture a compound represented by formula (III) (wherein R² represents -OCH(CH₃)COOR³ (R³ represents a hydrogen atom or a protecting group of a carboxyl group)) obtained in step (a') by washing with an aqueous solvent (preferably, an aqueous sodium hydrogencarbonate solution).

According to the present invention, a process for producing a compound represented by formula (I) is also provided. This process comprises step (c) of nitrating a compound represented by formula (IV) to give a mixture of a compound represented by formula (I) and a compound represented by formula (II) and step (d) of removing the compound represented by formula (II) from the mixture of the compound represented by formula (I) and the compound represented by formula (II). According to this process, a compound represented by formula (I), which is substantially free from a mutagen, can be produced.

In step (c), nitration can be carried out by using a reagent such as nitric acid, mixed acid, or metal nitrate salt. The product obtained by the nitration may also be used in the following step after crystallization.

In step (d), a compound represented by formula (II) can be selectively removed from a mixture of a compound represented by formula (I) and the compound represented by formula (II). Step (d) can be carried out in accordance with steps (a) and (b) or steps (a') and (b').

According to a preferred aspect of the present invention, there is provided a process for producing a compound represented by formula (I) (wherein R¹ is as defined above and Hal represents fluorine), comprising the steps of:
(c') nitrating a compound represented by formula (IV) (wherein R¹ is as defined above and Hal represents fluorine) to give a mixture of a compound represented by formula (I) and a compound represented by formula (II) (wherein Hal represents fluorine);
(a') reacting a compound represented by formula (II) with triethylamine and lactic acid optionally protected at a carboxyl group; and
(b') separating from the mixture a compound represented by formula (III) (wherein R² represents a group: -OCH(CH₃)COOR³ (R³ represents a hydrogen atom or a protecting group of carboxyl groups)) obtained in step (a') by washing with an aqueous solvent (preferably, an aqueous sodium hydrogencarbonate solution).

According to the present invention, a process for producing ME3127 is further provided. The process for producing ME3127 according to the present invention can be carried out in accordance with the following scheme.

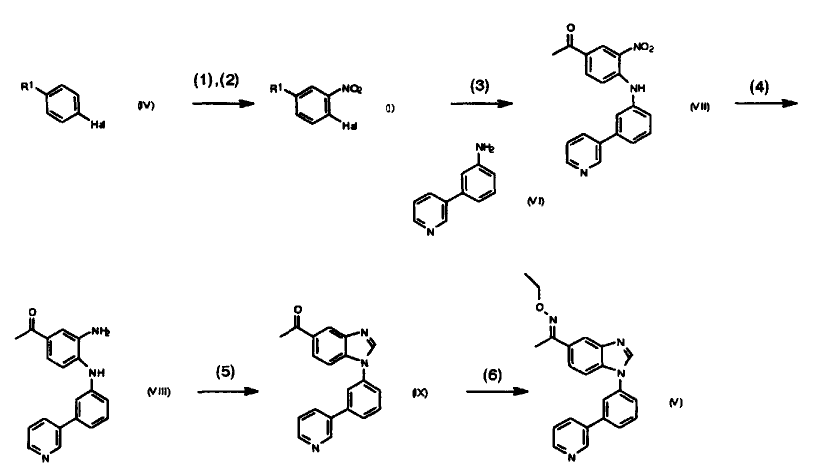

In steps (1) and (2), a compound represented by formula (I) is produced. When using a process for producing a compound represented by formula (I) according to the present invention, a compound represented by formula (I), which is substantially free from a compound represented by formula (II) as a mutagen, can be synthesized.

In step (1), nitration of a compound represented by formula (IV) can be carried out. Specifically, step (1) can be carried out in accordance with step (c), preferably step (c'). In step (2), the compound represented by formula (II) can be removed from a mixture of the compound represented by formula (I) and the compound represented by formula (II). Specifically, step (2) can be carried out in accordance with step (d) or steps (a) and (b), preferably steps (a') and (b').

The step of producing the compound represented by formula (I) can, preferably, comprise steps (c'), (a'), and (b').

In step (3), a compound represented by formula (I) can be bonded to a compound represented by formula (VI).

In step (4), a compound represented by formula (VII) can be reduced.

In step (5), a benzimidazole ring can be formed by subjecting a compound represented by formula (VIII) to a cyclization reaction.

In step (6), an acetyl group can be converted into CH₃CH₂ON=C(CH₃)-. This reaction can be carried out, for example, by heating and refluxing a compound represented by formula (IX) and o-ethylhydroxylamine hydrochloride in anhydrous ethanol.

Steps (3) to (6) can be carried out in accordance with the process described in WO96/33191.

When 2,4-dinitrofluorobenzene represented by formula (II) is formed in steps (1) and (2), the final compound is contaminated with three kinds of mutagens (XII) as described in the following scheme. Steps (3'), (4') and (5') each corresponds to steps (3), (4) and (5). 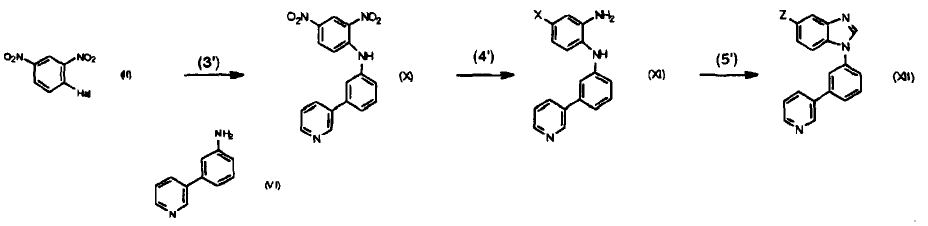 wherein, X represents NO₂ or NH₂, and Z represents NO₂, NH₂, or NHCHO.

As a result of HPLC analysis, it has been found that ME3127 bulks obtained in accordance with steps (1) to (6) are substantially free from a mutagen represented by formula (XII).

Accordingly, according to the process of the present invention, for example, ME3127 bulks free from the compound represented by formula (XII) derived from the compound represented by formula (II) can be produced.

### EXAMPLE

The present invention is further illustrated by the following Examples that are not intended as a limitation of the invention.

### Reference Example 1: Production of 4'-fluoro-3'-nitroacetophenone

4'-Fluoroacetophenone (33 g) was added dropwise to a mixed solution, composed of 144 ml of concentrated sulfuric acid and 56 ml of fuming nitric acid, cooled at -10 °C, and a reaction was allowed to proceed at that temperature for 2 hr. The reaction mixture was poured into 600 g of ice. The precipitated crystal was extracted with 400 ml of ethyl acetate, washed three times with 200 ml of water cooled at 5 °C, and washed twice with 100 ml of saturated saline. The ethyl acetate solution was concentrated to 50 ml, 50 ml of isopropyl alcohol was added to the concentrate, the solution was again concentrated to 50 ml, and the precipitated crystal (29.0 g) was collected by filtration.

The content of 2,4-dinitrofluorobenzene in the crystal was quantitatively determined by HPLC and found to be 1.4%.

### Example 1

4'-Fluoro-3'-nitroacetophenone (1.83 g) produced in Reference Example 1 was dissolved in 10 ml of acetonitrile. Benzyl alcohol (1.1 g) and 2.1 ml of triethylamine were added to the solution, followed by stirring at 30 °C for 1.5 hr. The reaction mixture was analyzed by HPLC. As a result, it was found that a peak derived from 2,4-dinitrofluorobenzene disappeared and, instead, a peak derived from 2,4-dinitrophenyl benzyl ether was newly detected.

### Example 2

4'-Fluoro-3'-nitroacetophenone (1.83 g) produced in Reference Example 1 was dissolved in 10 ml of acetonitrile. Anise alcohol (1.4 g) and 2.1 ml of triethylamine were added to the solution, followed by stirring at 30 °C for 1.5 hr. The reaction mixture was analyzed by HPLC. As a result, it was found that a peak derived from 2,4-dinitrofluorobenzene disappeared and, instead, a peak derived from 2,4-dinitrophenyl p-methoxybenzyl ether was newly detected.

### Example 3

4'-Fluoro-3'-nitroacetophenone (1.83 g) produced in Reference Example 1 was dissolved in 10 ml of acetonitrile. Isopropyl alcohol (0.6 g) and 2.1 ml of triethylamine were added to the solution, followed by stirring at 30 °C for 1.5 hr. The reaction mixture was analyzed by HPLC. As a result, it was found that a peak derived from 2,4-dinitrofluorobenzene disappeared and, instead, a peak derived from 2,4-dinitroisopropoxybenzene was newly detected.

### Example 4

4'-Fluoro-3'-nitroacetophenone (1.83 g) produced in Reference Example 1 was dissolved in 10 ml of acetonitrile. t-Butyl alcohol (0.7 g) and 2.1 ml of triethylamine were added to the solution, followed by stirring at 30 °C for 1.5 hr. The reaction mixture was analyzed by HPLC. As a result, it was found that a peak derived from 2,4-dinitrofluorobenzene disappeared and, instead, a peak derived from 2,4-dinitro-t-butoxybenzene was newly detected.

### Example 5

4'-Fluoro-3'-nitroacetophenone containing 0.84% of 2,4-dinitrofluorobenzene (1.83 g) was dissolved in 10 ml of acetonitrile. Acetic acid (0.15 g) and 0.7 ml of triethylamine were added to the solution, followed by stirring at 30 °C for 1.5 hr. The reaction mixture was analyzed by HPLC. As a result, it was found that a peak derived from 2,4-dinitrofluorobenzene disappeared and, instead, a peak derived from 2,4-dinitrophenyl acetate was newly detected.

### Example 6

4'-Fluoro-3'-nitroacetophenone (1.83 g) containing 0.84% of 2,4-dinitrofluorobenzene was dissolved in 10 ml of acetonitrile. Phenol (0.233 g) and 0.7 ml of triethylamine were added to the solution, followed by stirring at 30 °C for 1.5 hr. The reaction mixture was analyzed by HPLC. As a result, it was found that a peak derived from 2,4-dinitrofluorobenzene disappeared and, instead, a peak derived from 2,4-dinitrophenyl phenyl ether was newly detected.

### Example 7

4'-Fluoro-3'-nitroacetophenone (9.15 g) containing 0.8% of 2,4-dinitrofluorobenzene was dissolved in 20 ml of acetonitrile. Isopropyl alcohol (3.6 g) and 7.0 ml of triethylamine were added to the solution, followed by stirring at 30 °C overnight. The reaction mixture was analyzed by HPLC. As a result, it was found that a peak derived from 2,4-dinitrofluorobenzene disappeared and, instead, a peak derived from 2,4-dinitroisopropoxybenzene was newly detected. The reaction mixture was evaporated to dryness. The residue was dissolved in 20 ml of ethyl acetate and 20 ml of isopropyl alcohol. The solution was concentrated to about 9 ml, and the precipitated crystal (6.32 g) was collected by filtration.

A peak derived from 2,4-dinitrofluorobenzene in the crystal was not detected by HPLC. The recovery of 4'-fluoro-3'-nitroacetophenone was 69.0%, and a peak derived from 2,4-dinitroisopropoxybenzene was not detected.

### Example 8

4'-Fluoro-3'-nitroacetophenone (1.83 g) produced in Reference Example 1 was dissolved in 20 ml of acetonitrile. Lactic acid (0.9 g) and 0.7 ml of triethylamine were added to the solution, followed by stirring at 30 °C overnight. The reaction mixture was analyzed by HPLC. As a result, it was found that a peak derived from 2,4-dinitrofluorobenzene disappeared and, instead, a peak derived from o-(2,4-dinitrophenyl)lactic acid was newly detected. The reaction mixture was evaporated to dryness, and the residue was dissolved in 20 ml of ethyl acetate. The solution was washed twice with 5 ml of a saturated aqueous sodium hydrogencarbonate solution and then washed with 5 ml of saturated saline and evaporated to dryness (yield 1.35 g).

The content of 2,4-dinitrofluorobenzene in the residue was determined by LC/MS and found to be not more than 2 ppm. The recovery of 4'-fluoro-3'-nitroacetophenone was 73.9%, and a peak derived from o-(2,4-dinitrophenyl)lactic acid was not detected.

### Example 9

4'-Fluoro-3'-nitroacetophenone (1.83 g) produced in Reference Example 1 was dissolved in 20 ml of ethyl acetete. Lactic acid (0.9 g) and 0.7 ml of triethylamine were added to the solution, followed by stirring at 30 °C overnight. The reaction mixture was analyzed by HPLC. As a result, it was found that a peak derived from 2,4-dinitrofluorobenzene disappeared and, instead, a peak derived from o-(2,4-dinitrophenyl)lactic acid was newly detected. The reaction mixture was washed twice with 5ml of a saturated aqueous sodium carbonate solution and then washed with 5ml of saturated saline, and evaporated to dryness (yield 1.76 g).

The content of 2,4-dinitrofluorobenzene in the residue was determined by LC/MS and found to be not more than 2 ppm. The recovery of 4'-fluoro-3'-nitroacetophenone was 96.1%, and a peak derived from o-(2,4-dinitrophenyl)lactic acid was not detected.

### Example 10

4'-Fluoro-3'-nitroacetophenone (1.83 g) containing 3.3 % of 2,4-dinitrofluorobenzene was dissolved in 20 ml of ethyl acetete. Lactic acid (0.9 g) and 1.4 ml of triethylamine were added to the solution, followed by stirring at 30 °C overnight. The reaction mixture was analyzed by HPLC. As a result, it was found that a peak derived from 2,4-dinitrofluorobenzene disappeared and, instead, a peak derived from o-(2,4-dinitrophenyl)lactic acid was newly detected. The reaction mixture was washed twice with 5 ml of a saturated aqueous sodium hydrogencarbonate solution and then washed with 5 ml of saturated saline and evaporated to dryness (yield 1.73 g).

The content of 2,4-dinitrofluorobenzene in the residue was determined by LC/MS and found to be not more than 2 ppm. The recovery of 4'-fluoro-3'-nitroacetophenone was 94.5%, and a peak derived from o-(2,4-dinitrophenyl)lactic acid was not detected.

### Example 11

4'-Fluoro-3'-nitroacetophenone (1.83 g) produced in Reference Example 1 was dissolved in 20 ml of ethyl acetete. Lactic acid (0.9 g) and 1.4 ml of triethylamine were added to the solution, followed by stirring at 50 °C overnight. The reaction mixture was analyzed by HPLC. As a result, it was found that a peak derived from 2,4-dinitrofluorobenzene disappeared and, instead, a peak derived from o-(2,4-dinitrophenyl)lactic acid was newly detected. The reaction mixture was washed twice with 5 ml of a saturated aqueous sodium hydrogencarbonate solution and then washed with 5 ml of saturated saline and evaporated to dryness (yield 1.73 g).

The content of 2,4-dinitrofluorobenzene in the residue was determined by LC/MS and found to be not more than 2 ppm. The recovery of 4'-fluoro-3'-nitroacetophenone was 94.7%, and a peak derived from o-(2,4-dinitrophenyl)lactic acid was not detected.

## Claims

1. A process for removing a compound represented by formula (II): 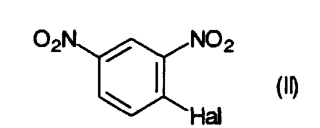 wherein Hal represents a halogen atom, from a mixture of a compound represented by formula (I): 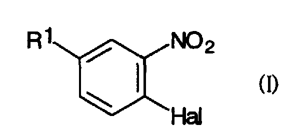 wherein R¹ represents a lower alkyl group, a lower alkylcarbonyl group, or a lower alkoxycarbonyl group, and Hal represents a halogen atom, and the compound represented by formula (II), comprising the steps of:
(a) reacting the compound represented by formula (II) with a tertiary amine and a nucleophilic reagent; and
(b) removing from the mixture a compound represented by formula (III) resulting in step (a): 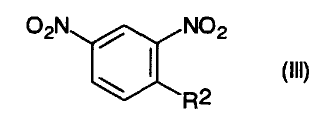 wherein R² represents a residue of the nucleophilic reagent.

2. The process according to claim 1, wherein the mixture of the compound represented by formula (I) and the compound represented by formula (II) is present in an organic solvent.

3. The process according to claim 1 or 2, wherein the removal is carried out by washing and/or crystallization.

4. The process according to claim 3, wherein the washing is carried out by using an aqueous solvent.

5. The process according to any one of claims 1 to 4, wherein the tertiary amine is triethylamine.

6. The process according to any one of claims 1 to 5, wherein the nucleophilic reagent is lactic acid, isopropyl alcohol, t-butyl alcohol, anise alcohol, or acetic acid.

7. The process according to any one of claims 1 to 6, wherein the compound represented by formula (II) is completely removed from the mixture.

8. A process for producing a compound represented by formula (I): 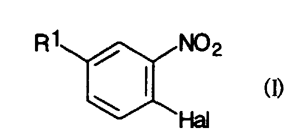 wherein R¹ and Hal are as defined in claim 1, comprising the steps of:
(c) nitrating a compound represented by formula (IV): 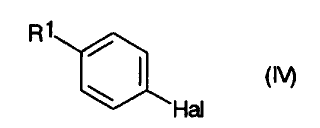 wherein R¹ and Hal are as defined in claim 1, to give a mixture containing a compound represented by formula (I) and a compound represented by formula (II): 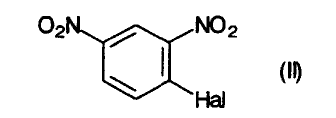 wherein Hal represents a halogen atom; and
(d) removing the compound represented by formula (II) from the mixture of the compound represented by formula (I) and the compound represented by formula (II) by using the process of any one of claims 1 to 7.

9. The process according to claim 8, wherein the final product is free from the compound represented by formula (II).

10. A process for producing a compound represented by formula (V): 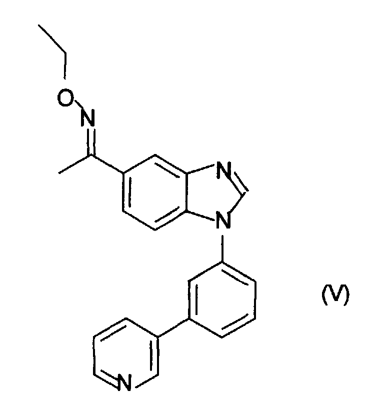 comprising the steps of:
producing a compound represented by formula (I) by using the process of claim 8 or 9;
reacting the compound represented by formula (I) with a compound represented by formula (VI) 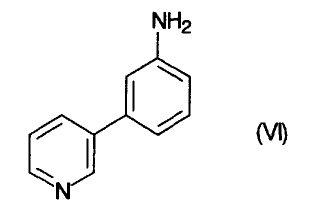 to produce a compound represented by formula (VII); 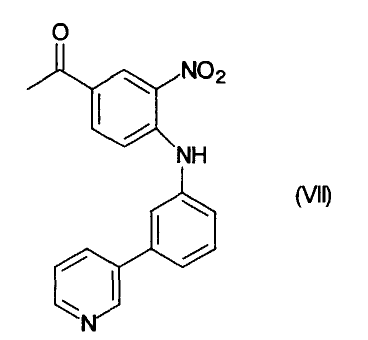
forming a benzimidazole ring in the compound represented by formula (VII); and
converting an acetyl group of formula (VII) into CH₃CH₂ON=C(CH₃)-.

11. The process according to claim 10, wherein the final product is free from the compound represented by formula (XII): 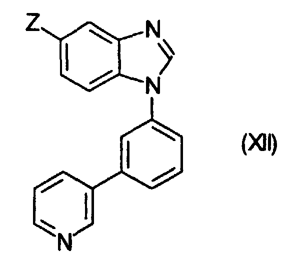 wherein Z represents NO₂, NH₂, or NHCHO.

12. A composition comprising a compound represented by formula (I), which is free from a compound represented by formula (II):

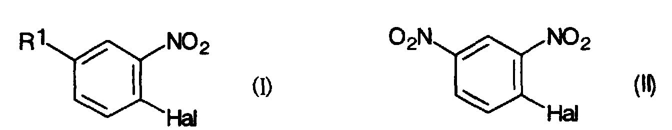

13. A composition comprising a compound represented by formula (V), which is free from a compound represented by formula (XII): 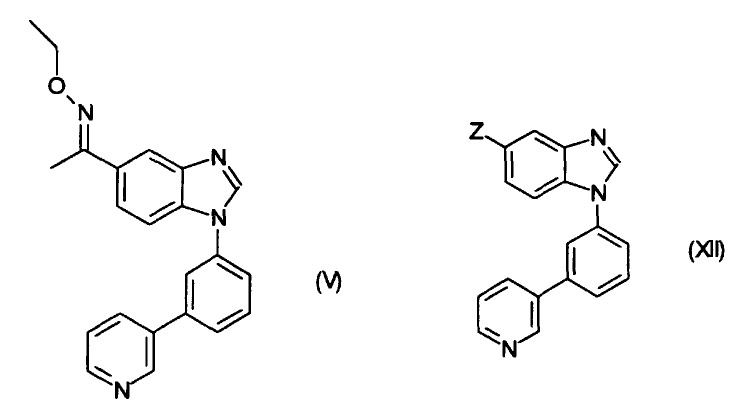 wherein Z represents NO₂, NH₂, or NHCHO.
